# EUROPÄISCHE PATENTSCHRIFT

(11) **EP 1 518 585 B1**
(45) Veröffentlichungstag und Bekanntmachung des Hinweises auf die Patenterteilung: **18.04.2007**
(21) Anmeldenummer: 04022843.9
(22) Anmeldetag: 24.09.2004
(51) Int. Cl.: A61N 5/06

(54) **Medizinisch-therapeutisches Bestrahlungsgerät mit einer Mehrzahl von Leuchtmitteln**
Medical therapeutical radiation treatment apparatus using a plurality of light sources
Appareil médical thérapeutique pour le traitement d'irradiation avec plusieurs sources de lumière

(30) Priorität: 24.09.2003 DE 20314801 U; 17.10.2003 DE 20315571 U
(43) Veröffentlichungstag der Anmeldung: 30.03.2005
(73) Patentinhaber: Kiontke, Siegfried, Dr., 82064 Strasslach (DE)
(72) Erfinder: Kiontke, Siegfried, Dr., 82064 Strasslach (DE)
(74) Vertreter: Winter, Brandl, Fürniss, Hübner Röss, Kaiser, Polte Partnerschaft Patent- und Rechtsanwaltskanzlei

(56) Entgegenhaltungen:
- EP-A- 0 674 468
- DE-A- 4 143 168
- US-A- 4 930 504
- US-A- 6 019 482
- US-A1- 2002 029 071
- US-B1- 6 537 305
- US-B1- 6 602 275

## Beschreibung

Die Erfindung betrifft ein medizinisch-therapeutisches Bestrahlungsgerät mit einer Mehrzahl von Leuchtmitteln nach dem Oberbegriff des Anspruchs 1.

Medizinisch-therapeutische Bestrahlungsgeräte die Licht im sichtbaren, im infraroten und im nahen Ultravioletbereich ausstrahlen, sind in einer Vielzahl von Ausgestaltungen bekannt.

Aus US-PS 4,535,784 ist eine Vorrichtung zur Stimulierung von Akupunkturpunkten durch Lichtstrahlung im sichtbaren Lichtbereich oder im Infrarotbereich bekannt. Aus US-PS 5,024,236 ist eine Fotosondenvorrichtung bekannt, die eine elektrische Sonde zur Auffindung von Akupunkturpunkten und eine Leuchtdiode zur Stimmulierung von Akupunkturpunkten sowie von verletzten Körperstellen umfaßt. Es wird eine einzige Diode verwendet.

Das französische Patent Nr. 2,591,902 offenbart ein Gerät zur Behandlung von Arthritis, bei dem ein Saugnapf, der eine Vielzahl von gepulsten Laserdioden enthält, auf dem Behandlungsbereich angebracht wird. Auf einer mit Konturen versehenen Oberfläche variiert der Abstand der Dioden von der Hautoberfläche. Zudem wird die Lichtenergie nicht gleichmäßig und konsistent aufgebracht. Aus dem französischen Patent Nr. 2,371,935 ist ein Bestrahlungsgerät bekannt, das gepulstes Infrarotlicht zu einer akupunkturähnlichen Behandlung erzeugt.

Das US-PS 3,900,034 offenbart die Verwendung einer Laser-Fotodiode zur Nervenstimmulierung, die Laserlicht in einem Bereich von Infrarot bis Ultraviolett aussendet. Sichtbares Licht nahe dem Infrarotbereich wird bevorzugt. Es wird ein Farbfleck auf dem Nerv verwendet um ihn zur Absorption der spezifischen Lichtwellenlänge, die von der Fotodiode erzeugt werden zu veranlassen. Das Licht ist gepulst.

Die sovjetischen Patente Nr. 1,266,540 und Nr. 1,289,493 lehren beide die Verwendung von mehreren Dioden, die senkrecht zur Haut angeordnet sind. Bei dem Patent Nr. 1,266,540 wird gepulste Infrarot-Strahlung verwendet. Ein Block von Dioden in Minigehäusen wird mit Klebeband oder Pflaster an dem zu bestrahlenden Bereich befestigt.

Aus UK-B 2,208,803 ist ein Gerät zur Applikation von Lichtimpulsen Akupunkturpunkte bekannt, wobei gepulstes weißes Licht durch einen Filter einer gewünschten Farbe oder durch eine dünne Schicht eines Medikaments projiziert wird.

Aus US-PS 4,232,678 ist ein Lichtakupunkturgerät bekannt, das eine Infrarotdiode mit variabler Frequenz für Akupunktur und Aurikolotherapie aufweist.

Aus US-PS 4,597,380 und US-PS 4,072,147 sind Endoskope bekannt, die Laserlicht an eine Operationsstelle im Körper abgeben. Aus US-PS 4,693,556, aus US-PS 5,059,191 und aus US-PS 4,998,930 sind jeweils Bestrahlungsgeräte bekannt, die rotes Licht verwenden, das von optischen Fasern zu einer inneren Stelle im menschlichen Körper geleitet wird um Krebszellen oder andere Tumore zu zerstören.

Aus der EP-B-0 741 594 ist ein Bestrahlungsgerät bekannt, das eine Mehrzahl von Leuchtmitteln in Matrixform aufweist, die eine Applikationsfläche aufspannen. Die Mehrzahl der LEDs strahlen Licht im roten bis infraroten Wellenlängenbereich aus. Zusätzlich kann mit dem Gerät durch eine Heizvorrichtung Wärme auf die Behandlungsfläche aufgebracht werden.

Aus den Druckschriften US 4,930,504 B, EP 674 468 A2, US 4,232,678 B, EP 741 594 B1 und WO 93/09847 sind weitere optisch-medizinische Bestrahlungsgeräte mit LEDs bekannt.

Aus der US 2002/0029071 A1 ist ein medizinisch-therapeutisches Bestrahlungsgerät mit einer Mehrzahl von Leuchtmitteln in Matrixform bekannt, wobei die Mehrzahl der Leuchtmittel in mehrere Leuchtmittelgruppen unterteilt sind, die Licht unterschiedlicher Wellenlängenbereiche ausstrahlen. Die Wellenlängebereiche umfassen breitbandiges Infrarotlicht zur Wärmebehandlung sowie schmalbandiges Licht im roten, grünen und blauen Wellenlängenbereich des sichtbaren Lichts zur fotdynamischen Therapie.

Schließlich ist aus der DE 41 08 328 A1 ein medizinisch-therapeutisches Bestrahlungsgerät mit einer Mehrzahl von Leuchtmitteln in Matrixform bekannt, wobei die Mehrzahl der Leuchtmittel in mehrere Leuchtmittelgruppen unterteilt sind, die Licht unterschiedlicher Wellenlängenbereiche ausstrahlen und die selektiv aktivierbar sind. Die Wellenlängebereiche umfassen breitbandiges Infrarotlicht zur Wärmebehandlung sowie UV-Licht und sichtbares Licht.

Aus der US-B-6 602 275, der DE 41 43 168 A und der US-A-6 019 482 ist gegenüber der DE 41 08 328 A1 zusätzlich das Vorsehen einer Leuchtmittelgruppe für monochromes, schmalbandiges Infrarotlicht, einer Leuchtmittelgruppe für monochromes, schmalbandiges rotes Licht und einer Leuchtmittelgruppe für monochromes, schmalbandiges blaues Licht bekannt.

Ausgehend von einer der drei letztgenannten Druckschriften ist es Aufgabe der vorliegenden Erfindung ein medizinisch-therapeutisches Bestrahlungsgerät anzugeben, das eine Vielzahl von Behandlungsvarianten ermöglicht und eine erhöhte Wirksamkeit besitzt.

Die Lösung dieser Aufgabe erfolgt durch die Merkmale des Anspruchs 1.

Dadurch, daß sich die Leuchtmittelgruppen selektiv aktivieren lassen, können sie einzeln oder in beliebigen Gruppen zusammen zur Bestrahlung eingesetzt werden. Damit kann für den jeweils gewünschten Anwendungszweck die jeweils wirksamste Kombination von Wellenlängenbereichen ausgewählt werden. Durch die Verwendung von LEDs mit schmalbandigem Infrarotlicht werden Wachstums- und Wundheilungsprozesse positiv beeinflußt. Die Verwendung von Infrarotstrahlung zu medizinischen Zwecken ist seit langem bekannt. Bei der Absorption von IR-Strahlung erhöht sich die Bewegungsenergie der Moleküle. Im lebenden Gewebe hat diese Erhöhung beschleunigenden Einfluß auf den Verlauf biochemischer Reaktionen und auf die Zirkulation (Durchblutung) und somit Sauerstoffversorgung des Gewebes. Bisher wurde in der Medizin davon ausgegangen, daß rotes bzw. infrarotes Licht nur eine geringe physiologische Relevanz besitzt. Diese Meinung mußte aufgrund von Experimenten an Bord sowohl russischer als auch amerikanischer Raumstationen revidiert werden. Es wurden dort zusätzlich beschleunigende Wirkungen auf Wachstum und Wundheilungsprozesse gefunden, die nicht von der Intensität der jeweiligen Infrarotstrahlung, sondern lediglich von der jeweiligen Wellenlänge abhängig waren.

Durch das Vorsehen einer Leuchtmittelgruppe für monochromes, schmalbandiges infrarotes Licht (zweite Infrarotgruppe) mit einer Peakwellenlänge von 754 nm und einer Leuchtmittelgruppe für monochromes, schmalbandiges blaues Licht (Blaugruppe) mit einer Peakwellenlänge von 457 nm ergibt sich eine verbesserte Wirksamkeit im Zusammenwirken mit der ersten Infrarotgruppe.

Die bisher bekannt Geräte für Flächenbestrahlung, die Infrarotstrahlung einsetzen, verwenden ein hauptsächlich breitbandiges Spektrum, d. h. es wird auf die thermische Wirkung abgestellt. Zwar sind aus dem Lichtakupunkturbereich Geräte bekannt, z.B. US-PS 4,232,678, US 5,259,380 B oder der GB 2 212 010 A, die schmalbandige variable Infrarotwellenlängen verwenden. Hierbei wird jedoch das schmalbandige Infrarotlicht nicht flächig auf die zu behandelnde Fläche aufgetragen, sondern mittels fokussierenden Reflektoren, auch bedingt durch den Anwendungszweck Akupunktur, ganz gezielt auf bestimmte räumlich eng begrenzte Hautpunkte.

Gemäß einer bevorzugten Ausgestaltung der Erfindung nach den Ansprüchen 2 und 3, strahlt die erste Infrarotgruppe Infrarotlicht in Wellenlängenbereich zwischen 850 und 960 nm aus. Die Peakwellenlänge beträgt hierbei 950 nm. Bei Anwendungsbeobachtungen als Voraussetzung für die Zertifizierung hat sich herausgestellt, daß dieser schmale Wellenlängenbereich besonders wirksam ist.

Gemäß der bevorzugten Ausgestaltung der Erfindung nach Anspruch 4 strahlt die zweite Infrarotgruppe infrarotes Licht im Wellenlängenbereich zwischen 750 und 770 nm bei einer Peakwellenlänge von 754 nm aus. Auch dieser Wellenlängenbereich hat sich als besonders wirksam erwiesen.

Gemäß einer weiteren bevorzugten Ausgestaltung der Erfindung nach den Ansprüchen 5 bis 7 umfassen die Leuchtmittelgruppen eine Grüngruppe, die Licht im grünen sichtbaren Bereich des Spektrums und zwar Wellenlängen zwischen 520 bis 570 nm mit einer Peakwellenlänge von 540 nm ausstrahlen. Auch dieser schmale Wellenlängenbereich hat sich als besonders wirksam erwiesen. Grünes Licht ist nicht nur für die Fotosynthese grüner Pflanzen relevant, sondern stimuliert auch die Mitochondrien verletzter Hautzellen und beschleunigt somit den Wundheilungsprozess.

Gemäß einer bevorzugten Ausgestaltung der Erfindung nach Anspruch 8 strahlt die Blaugruppe blaues bzw. blauviolettes Licht im sichtbaren Wellenlängenbereich mit einer Wellenlänge von 400 bis 470 nm und einer Peakwellenlänge von 457 nm aus. Auch dieser Wellenlängenbereich hat sich als besonders wirksam erwiesen. Durch Untersuchungen von Dr. Popp an Benzo(a)-bzw. Benzo(e)-Pyren ist bekannt, daß blauviolettes Licht Reparaturen an beschädigten Chromosomen vornehmen kann. Diese als Fotoreparatur bekannte Tatsache ist auch im Bereich chronischer Erkrankungen mit sehr gutem Erfolg einsetzbar.

Ein weiterer Vorteil der vorgenannten Wellenlängenbereiche im grünen, blauen und infraroten schmalbandigen Wellenlängenbereich besteht darin, daß es hierfür LEDs gibt, die standardmäßig zu erwerben sind; es sind daher keine teuren Spezialanfertigungen notwendig.

Aufgrund der speziell gewählten infraroten, grünen und blauvioletten schmalen Frequenzbereiche hat sich das erfindungsgemäße Bestrahlungsgerät als wirksam bei der Heilung unterschiedlicher Beschwerden bzw. Erkrankungen gezeigt.

Gemäß einer vorteilhaften Ausgestaltung der Erfindung nach Anspruch 9 wird diese Wirkung noch dadurch unterstützt bzw. verstärkt, daß ein Pulsbetrieb mit verschiedenen Frequenzen vorgesehen werden kann. Für die Pulsfrequenzen haben sich insbesondere Frequenzen von 1,85 Hz, 5,03 Hz, 7,8 Hz, 13,7 Hz, 37,2 Hz, 101 Hz, 275 Hz, 747 Hz, 2031 Hz, 5522 Hz, 15011 Hz, und/oder 40804 Hz als besonders wirksam herausgestellt.

Gemäß einer bevorzugten Ausgestaltung der Erfindung nach Anspruch 11 liegt die Strahlungsleistung der einzelnen Leuchtmittel im Bereich zwischen 1 und 10 Watt. Durch diese sehr geringe Strahlungsleistung werden schädliche Wirkungen zu hohe Strahlungsleistung ausgeschlossen und es wird noch einmal deutlich, daß die Wirkung des erfindungsgemäßen Bestrahlungsgeräts durch die Kombination der unterschiedlichen schmalen Wellenlängenbereich gegeben ist.

Durch die bevorzugte Ausgestaltung der Erfindung nach Anspruch 12 lassen sich über Bedienungselemente und dem Steuermittel unterschiedliche Behandlungsprogramme aktivieren. Diese Behandlungsprogramme, die auch nachträglich in das Gerät installiert werden können, können für verschiedene Behandlungszwecke und für verschiedene Patienten besonders wirksame Wellenlängebereiche mit bestimmter Dauer und/oder bestimmten Pulsfrequenzen ausgewählt werden.

Durch die bevorzugte Ausgestaltung nach Anspruch 13 sind sowohl die Leuchtmittel als auch die Steuermittel und Bedienungselemente in einem gemeinsamen Gehäuse angeordnet und stellen damit ein kompaktes Gerät zur Verfügung das auch für die Anwendung durch den Laien geeignet ist.

Weitere Einzelheiten, Merkmale und Vorteile der Erfindung ergeben sich aus der nachfolgenden Schreibung einer bevorzugten Ausführungsform der Erfindung anhand der Zeichnung.

### Es zeigt:

Fig. 1 zeigt eine Aufsicht auf eine beispielhafte Ausführungsform der Erfindung mit den in Matrixform angeordneten LEDs und Bedienungselementen,
Fig. 2 eine perspektivische Darstellung der in Fig. 1 gezeigten Ausführungsform.

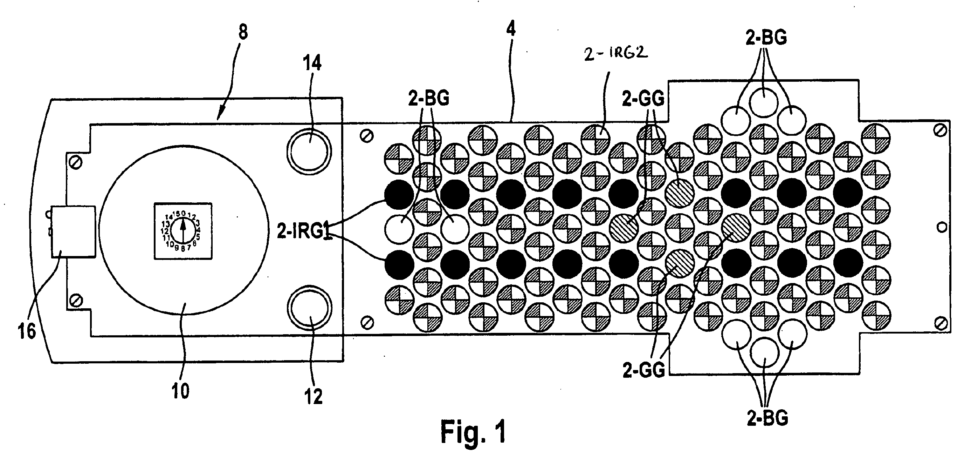

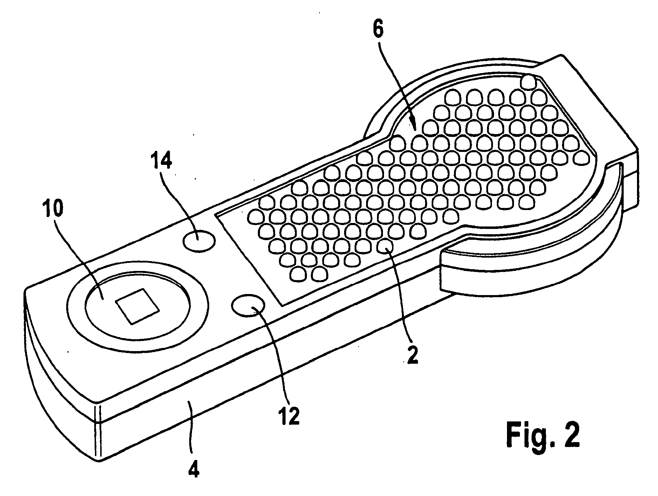

Die in den Fig. 1 und 2 gezeigte beispielhafte Ausführungsform der Erfindung umfaßt eine Mehrzahl von Leuchtmitteln in Form von LEDs 2 die in einem gemeinsamen Gehäuse 4 in Matrixform angeordnet sind und eine Aplikationsfläche 6 aufspannen. Die einzelnen LEDs 2 sind in vier Leuchtmittelgruppen, nämlich eine erste Infrarotgruppe IRG1, eine zweite Infrarotgruppe IRG2, eine Grüngruppe GG und eine Blaugruppe BG unterteilt. Neben der Aplikationsfläche 6 ist ein Bedienungsfeld 8 vorgesehen, das einen Programmwahlschalter 10, eine Starttaste 12 und eine Stopptaste 14 umfaßt.

Die erste Infrarotgruppe IRG1 umfaßt 16 Infrarot-LEDs 2-IRG1, die in Fig. 1 schwarz gefärbt dargestellt sind. Die Grüngruppe GG umfaßt vier grünfarbige LEDs 2-GG die im mittleren Bereich des Aplikationsfläche 6 angeordnet sind und querschrafiert dargestellt sind. Die Blaugruppe BG umfaßt acht blaue LEDs 2-BG, die seitlich und in unteren mittigen Bereich der Applikationsfläche 6 im Anschluß an das Bedienungsfeld 8 angeordnet sind und als Kreise dargestellt sind. Die zweite Infrarotgruppe umfaßt 79 infrarote LEDs 2-IRG2 die mit einer Rotorschrafur versehen sind. Bei der beispielhaften Ausgestaltung umfaßt die Matrixanordnung 18 Zeilen mit jeweils abwechselnd fünf bzw. sechs LEDs. Lediglich die vierte Zeile umfaßt sechs LEDs, die siebte Zeile acht LEDs und die vierte Zeile wiederum sechs LEDs.

Die vier Leuchtmittelgruppen IRG1, IRG2, GG und BG strahlen jeweils Licht unterschiedlicher Wellenlängen in einem schmalbandigen Wellenlängenbereich aus. Die erste Infrarotgruppe IRG1 strahlt Infrarotlicht in einem Wellenlängenbereich zwischen 850 und 960 nm mit einer Peakwellenlänge von 950 nm aus. Die zweite Infrarotgruppe IRG2 strahlt Licht in einem Wellenlängenbereich zwischen 750 und 770 nm und einer Peakwellenlänge von 754 nm aus. Die Grüngruppe GG strahlt Licht in einem Wellenlängenbereich zwischen 520 und 570 nm aus mit einer Peakwellenlänge von 540 nm. Die Blaugruppe strahlt Licht in einem Wellenlängenbereich zwischen 400 und 470 nm mit einer Peakwellenlänge von 457 nm aus. Für die LEDs 2 können beispielsweise LEDs der Firma Fitie mit der jeweiligen Wellenlängecharakteristik verwendet werden. Die einzelnen LEDs 2 sind über ein nicht dargestelltes Steuermittel in Form einer Mikrocomputerschaltung mit den Bedienungselementen 8, 12 und 14 verbunden. Über eine Netzteilbuchse 16 wird das Bestrahlungsgerät mit Strom versorgt.

Mittels der Mikrocomputerschaltung sind 15 unterschiedliche Bestrahlungsprogramme realisiert, die sich hinsichtlich der verwendeten Pulsfrequenz und der zeitlichen Dauer der Bestrahlung voneinander unterscheiden.

Diese fünfzehn beispielhaften Programme sind nachfolgend aufgelistet:

| **Programm** | **Puls-Frequenz** | | |
|---|---|---|---|
| 0 | Alle LEDs aus | | |
| 1 | Alle LEDS ein (Dauerlicht) | | |
| 2 | 1,85 Hz | | |
| 3 | 5,03 Hz | | |
| 4 | 7,8 Hz | | |
| 5 | 12,7 Hz | | |
| 6 | 37,2 Hz | | |
| 7 | 101 Hz | | |
| 8 | 275 Hz | | |
| 9 | 747 Hz | | |
| 10 | 2032 Hz | | |
| 11 | Kombinationsablauf 1: | | |
| | a | 30 sec | Alle LEDs ein |
| | b | 1 min | 5,03 Hz |
| | c | 2 min | 13,7 Hz |
| | d | 30 sec | 2032 Hz |
| | Gesamt: 4min | | |
| 12 | Kombinationsablauf 2: | | |
| | a | 1 min | Alle LEDs ein |
| | b | 2 min | 5,03 Hz |
| | c | 1 min | 13,7 Hz |
| | d | 30 sec | 2032 Hz |
| 13 | Gesamt: 4 min 30 sec | | |
| | Kombinationsablauf 3: | | |
| | a | 2 min | Alle LEDs ein |
| | b | 30 sec | 5,03 Hz |
| | c | 1 min | 13,7 Hz |
| | d | 2 min | 2032 Hz |
| 14 | Gesamt: 5 min 30 sec | | |
| | Kombinationsablauf 4: | | |
| | a | 1 min 30 sec | 1,85 Hz |
| | b | 1 min 50 sec | 7.8 Hz |
| | c | 2 min 10 sec | 37,2 Hz |
| | d | 1 min 50 sec | 275 Hz |
| | e | 1 min 50 sec | 2032 Hz |
| 15 | Gesamt: 9 min 10 sec | | |
| | Kombinationsablauf 5: | | |
| | a | 2 min | 1,85 Hz |
| | b | 1 min 30 sec | 5,03 Hz |
| | c | 1 min 40 sec | 13,7 Hz |
| | d | 1 min 50 sec | 101 Hz |
| | e | 50 sec | 2032 Hz |
| | Gesamt: 7 min 50 sec | | |

In der Stellung "0" des Programmwalhschalters 10 sind alle LEDs 2 ausgeschaltet. Bei der Schalterstellung "1", d. h. dem Bestrahlungsprogramm 1, werden alle LEDs auf Dauerlicht geschaltet. Bei den Schalterstellungen "2" bis "10" werden die LEDs 2 jeweils mit unterschiedlichen Pulsfrequenzen gepulst.

Bei den Schalterstellungen "11" bis "15" handelt es sich um Kombinationsläufe bei denen zu verschiedenen Zeiten unterschiedliche Pulsfrequenzen verwendet werden. In Anwendungsbeobachtungen hat sich herausgestellt, daß das Programm "11" für hochakute Zustände, das Programm "12" für mittelchronische Zustände, das Programm "13" für regulationsstarre Patienten, das Programm "14" für Allergiebehandlung und das Programm "15" für Schmerzbehandlung besonders geeignet und wirksam ist.

Bei der vorstehend beschriebenene Ausführungsform werden bei allen Programmen jeweils alle LEDs aktiviert. Natürlich können auch Programme vorgesehen werden, bei denen nur bestimmte Leuchtmittelgruppen einzeln oder in Kombination miteinander aktiv sind.

## Patentansprüche

1. Medizinisch-therapeuthisches Bestrahlungsgerät mit einer Mehrzahl von Leuchtmitteln, insbesondere in Form von LEDs (2), die in einem Gehäuse (4) in Matrixform angeordnet sind und eine Applikationsfläche (6) aufspannen,
wobei die Mehrzahl von Leuchtmittel in eine Mehrzahl von Leuchtmittelgruppen (IRG1, IRG2, GG, BG) unterteilt sind, die Licht unterschiedlicher Wellenlängebereiche ausstrahlen,
wobei die Mehrzahl der Leuchtmittelgruppen (IRG1, IRG2, GG, BG) wenigstens eine erste Infrarotgruppe (IRG1) für monochromes, schmalbandiges Infrarotlicht, wenigstens eine zweite Infrarotgruppe (IRG2) für monochromes, schmalbandigen infrarotes Licht und wenigstens eine Blaugruppe (BG) für monochromes, schmalbandigen blaues Licht umfassen, und
wobei die einzelnen Leuchtmittelgruppen (IRG1, IRG2, GG, BG) mittels einem Steuermittel selektiv aktivierbar sind,
**dadurch gekennzeichnet,**
**dadurch gekennzeichnet, dass** die zweite Infrarotgruppe (IRG2) eine Peakwellenlänge von 754 nm und die Blaugruppe (BG) eine Peakwellenlänge von 457 nm aufweist.

2. Bestrahlungsgerät nach Anspruch 1, **dadurch gekennzeichnet, dass** die erste Infrarotgruppe (IRG1) Infrarotlicht in einem Wellenlängenbereich zwischen 850 und 960 nm ausstrahlt.

3. Bestrahlungsgerät nach Anspruch 1, 2 oder 3, **dadurch gekennzeichnet, dass** die erste Infrarotgruppe (IRG1) eine Peak-Wellenlänge von 950 nm aufweist.

4. Bestrahlungsgerät nach einem der vorhergehenden Ansprüche, **dadurch gekennzeichnet, dass** die zweite Infrarotgruppe (IRG2) infrarotes Licht in einem Wellenlängenbereich zwischen 750 und 770 nm ausstrahlt.

5. Bestrahlungsgerät nach einem der vorhergehenden Ansprüche, **dadurch gekennzeichnet, dass** die Mehrzahl der Leuchtmittelgruppen wenigstens eine Grüngruppe (GG) für monochromes, schmalbandiges grünes Licht umfaßt.

6. Bestrahlungsgerät nach Anspruch 5, **dadurch gekennzeichnet, dass** die Grüngruppe (GG) grünes Licht in einem Wellenlängenbereich zwischen 520 und 570 nm ausstrahlt.

7. Bestrahlungsgerät nach Anspruch 5 oder 6, **dadurch gekennzeichnet, dass** die Grüngruppe (GG) eine Peak-Wellenlänge von 540 nm aufweist.

8. Bestrahlungsgerät nach einem der vorhergehenden Ansprüche, **dadurch gekennzeichnet, dass** die Blaugruppe (BG) blaues Licht in einem Wellenlängenbereich zwischen 400 und 470 nm ausstrahlt.

9. Bestrahlungsgerät nach einem der vorhergehenden Ansprüche, **dadurch gekennzeichnet, dass** wenigstens eine der Mehrzahl der Leuchtmittelgruppen (IRG1, IRG2, GG, BG) gepulstes Licht abstrahlt.

10. Bestrahlungsgerät nach einem der vorhergehenden Ansprüche, **dadurch gekennzeichnet, dass** die Pulsfrequenz des gepulsten Lichts 1,85 Hz, 5,03 Hz, 7,8 Hz, 13,7 Hz, 37,2 Hz, 101 Hz, 275 Hz, 747 Hz, 2031 Hz, 5522 Hz, 15011 Hz und/oder 40804 Hz umfaßt.

11. Bestrahlungsgerät nach einem der vorhergehenden Ansprüche, **dadurch gekennzeichnet, dass** die Strahlungsleistung der einzelnen Leuchtmittel (2) im Bereich zwischen 1 und 10 W liegt.

12. Bestrahlungsgerät nach einem der vorhergehenden Ansprüche, **dadurch gekennzeichnet, dass** sich mittels Bedienungselementen, (10, 12, 14) die mit dem Steuermittel verbunden sind, unterschiedliche Bestrahlungsprogrammes aktivieren lassen.

13. Bestrahlungsgerät nach einem der vorhergehenden Ansprüche, **dadurch gekennzeichnet, dass** die Leuchtmittel (2), die Bedienungselemente (10, 12, 14) und das Steuermittel in dem gemeinsamen Gehäuse (4) angeordnet sind.

## Claims

1. Medical treatment radiation device with a plurality of light-emitting means, in particular in the form of LEDs (2), which are disposed in a matrix pattern in a housing (4) and span an application surface (6),
which plurality of light-emitting means is subdivided into a plurality of groups of light-emitting means (IRG1, IRG2, GG, BG) which emit light in different wavelength ranges,
and the plurality of groups of light-emitting means (IRG1, IRG2, GG, BG) comprises at least a first infrared group (IRG1) for monochromatic narrow-band infrared light, at least a second infrared group (IRG2) for monochromatic narrow-band infrared light and at least a blue group (BG) for monochromatic narrow-band blue light, and
the individual groups of light-emitting means (IRG1, IRG2, GG, BG) can be selectively activated by means of a control means,
**characterised in that**
the second infrared group (IRG2) has a peak wavelength of 754 nm and the blue group (BG) has a peak wavelength of 457 nm.

2. Radiation device as claimed in claim 1, **characterised in that** the first infrared group (IRG1) emits infrared light in a wavelength range between 850 and 960 nm.

3. Radiation device as claimed in claim 1, 2 or 3, **characterised in that** the first infrared group (IRG1) has a peak wavelength of 950 nm.

4. Radiation device as claimed in one of the preceding claims, **characterised in that** the second infrared group (IRG2) emits infrared light in a wavelength range between 750 and 770 nm.

5. Radiation device as claimed in one of the preceding claims, **characterised in that** the plurality of groups of light-emitting means includes at least one green group (GG) for monochromatic narrow-band green light.

6. Radiation device as claimed in claim 5, **characterised in that** the green group (GG) emits green light in a wavelength range between 520 and 570 nm.

7. Radiation device as claimed in claim 5 or 6, **characterised in that** the green group (GG) has a peak wavelength of 540 nm.

8. Radiation device as claimed in one of the preceding claims, **characterised in that** the blue group (BG) emits light in a wavelength range between 400 and 470 nm.

9. Radiation device as claimed in one of the preceding claims, **characterised in that** at least one of the plurality of groups of light-emitting mans (IRG1, IRG2, GG, BG) emits pulsed light.

10. Radiation device as claimed in one of the preceding claims, **characterised in that** the pulse frequency of the pulsed light is 1.85 Hz, 5.03 Hz, 7.8 Hz, 13.7 Hz, 37.2 Hz, 101 Hz, 275 Hz, 747 Hz, 2031 Hz, 5522 Hz, 15011 Hz and/or 40804 Hz.

11. Radiation device as claimed in one of the preceding claims, **characterised in that** the radiation intensity of the individual light-emitting means (2) is in the range of between 1 and 10 Watt.

12. Radiation device as claimed in one of the preceding claims, **characterised in that** different radiation programmes can be activated by means of operating elements (10, 12, 14) connected to the control means.

13. Radiation device as claimed in one of the preceding claims, **characterised in that** the light-emitting means (2), the operating elements (10, 12, 14) and the control means are disposed in a common housing (4).

## Revendications

1. Appareil d'irradiation médico-thérapeutique avec une pluralité de moyens lumineux, en particulier sous la forme de DEL (2), qui sont disposés dans un boîtier (4) sous forme de matrice et s'étendent sur une surface d'application (6),
la pluralité de moyens lumineux étant subdivisés en une pluralité de groupes de moyens lumineux (IRG1, IRG2, GG, BG), qui diffusent une lumière de diverses plages de longueurs d'onde,
la pluralité de groupes de moyens lumineux (IRG1, IRG2, GG, BG) comprenant au moins un premier groupe infrarouge (IRG1) pour une lumière infrarouge monochrome à brande étroite, au moins un second groupe infrarouge (IRG2) pour une lumière infrarouge monochrome à bande étroite et au moins un groupe bleu (BG) pour une lumière bleue monochrome à bande étroite, et
chacun des groupes de moyens lumineux (IRG1, IRG2, GG, BG) étant activable de manière sélective à l'aide d'un moyen de commande,
**caractérisé en ce que** le second groupe infrarouge (IRG2) présente une longueur d'onde de pic de 754 nm et le groupe bleu (BG) présente une longueur d'onde de pic de 457 nm.

2. Appareil d'irradiation selon la revendication 1, **caractérisé en ce que** le premier groupe infrarouge (IRG1) émet une lumière infrarouge présentant une plage de longueurs d'onde comprise entre 850 et 960 nm.

3. Appareil d'irradiation selon la revendication 1, 2 ou 3, **caractérisé en ce que** le premier groupe infrarouge (IRG1) présente une longueur d'onde de pic de 950 nm.

4. Appareil d'irradiation selon l'une quelconque des revendications précédentes, **caractérisé en ce que** le second groupe infrarouge (IRG2) émet une lumière infrarouge présentant une plage de longueurs d'onde comprise entre 750 et 770 nm.

5. Appareil d'irradiation selon l'une quelconque des revendications précédentes, **caractérisé en ce que** la pluralité de groupes de moyens lumineux comprennent au moins un groupe vert (GG) pour une lumière verte monochrome à bande étroite.

6. Appareil d'irradiation selon la revendication 5, **caractérisé en ce que** le groupe vert (GG) émet une lumière verte présentant une plage de longueur d'onde comprise entre 520 et 570 nm.

7. Appareil d'irradiation selon la revendication 5 ou 6, **caractérisé en ce que** le groupe vert (GG) présente une longueur d'onde de pic de 540 nm.

8. Appareil d'irradiation selon l'une quelconque des revendications précédentes, **caractérisé en ce que** le groupe bleu (BG) émet une lumière bleue présentant une plage de longueur d'onde comprise entre 400 et 470 nm.

9. Appareil d'irradiation selon l'une quelconque des revendications précédentes, **caractérisé en ce qu'**au moins un groupe parmi la pluralité de groupes de moyens lumineux (IRG1, IRG2, GG, BG) émet une lumière pulsée.

10. Appareil d'irradiation selon l'une quelconque des revendications précédentes, **caractérisé en ce que** la fréquence d'impulsion de la lumière pulsée est de 1,85 Hz, 5,03 Hz, 7,8 Hz, 13,7 Hz, 37,2 Hz, 101 Hz, 275 Hz, 747 Hz, 2031 Hz, 5522 Hz, 15011 Hz et/ou 40804 Hz.

11. Appareil d'irradiation selon l'une quelconque des revendications précédentes, **caractérisé en ce que** la puissance de rayonnement de chaque moyen lumineux (2) se trouve dans une plage comprise entre 1 et 10 W.

12. Appareil d'irradiation selon l'une quelconque des revendications précédentes, **caractérisé en ce que** divers programmes d'irradiation peuvent être activés au moyen d'éléments de commande (10, 12, 14) reliés au moyen de commande.

13. Appareil d'irradiation selon l'une quelconque des revendications précédentes, **caractérisé en ce que** les moyens lumineux (2), les éléments de commande (10, 12, 14) et le moyen de commande sont disposés dans le boîtier commun (4).
